# EUROPEAN PATENT APPLICATION

(11) **EP 4 807 754 A1**
(43) Date of publication of application: **16.09.2026**
(21) Application number: 25168627.5
(22) Date of filing: 04.04.2025
(51) Int. Cl.: G16H 15/00, G16H 40/40, G16H 40/63, G16H 40/67, G16H 30/40, G16H 50/70

(54) **DEVICES AND METHODS FOR GENERATING POST-OPERATIONAL DATA OF AN IMAGING DEVICE**

(30) Priority: 13.03.2025 US 202563771482 P
(71) Applicant: Leica Microsystems CMS GmbH, 35578 Wetzlar (DE)
(72) Inventor: SERRA LLETI, Jose Miguel, 35578 Wetzlar (DE); FEHLAUER, Holger, 35578 Wetzlar (DE); CHOI, Won Yung, 35578 Wetzlar (DE); MCELROY, Sean, 35578 Wetzlar (DE)
(74) Representative: Paustian & Partner Patentanwälte mbB

(57) **Abstract**

A first aspect of the present disclosure is related to a device for generating post-operational data of an imaging device,
configured to:
- obtain operational data of an imaging device;
- determine instruction data based on:
-- the operational data; and
- determine a post-operational data set based on:
-- the instruction data; and
-- the operational data.

## Description

### Technical Field

This disclosure is related to devices and methods for generating post-operational data of an imaging device, such as a microscope.

### Background

Medical experts routinely perform imaging with imaging devices, such as microscopes, then analyze multiple images to uncover findings that may trigger further experiments. As part of this workflow, they often use specialized image analysis software to detect, segment, and phenotype objects of interest, ultimately extracting meaningful results from quantitative measurements. Experts have to rely on multiple platforms, such as Word, Excel, or PowerPoint, to assemble reports on their imaging and analysis sessions, which is both labor-intensive and time-consuming. Moreover, there is no straightforward way to feedback findings based on operational data into an operational workflow or to another device. This prevents users from effectively adjusting imaging devices and/or analysis setups based on new insights. Improvements are desirable.

### Summary

An object of the present disclosure is to improve working with an imaging device.

This object is solved by the disclosed embodiments, which are defined in particular by the subject matter of the independent claims. The dependent claims provide information for further embodiments. Various aspects and embodiments of these aspects are also disclosed in the summary and description below, which provide additional features and advantages.

A first aspect of the present disclosure is related to a device for generating post-operational data of an imaging device,
*configured to:*
- *obtain operational data of an imaging device;*
- *determine instruction data based on:*
   -- *the operational data; and*
- *determine a post-operational data set based on:*
   -- *the instruction data; and*
   -- *the operational data.*

An image device is configured for image acquisition. **It** can be a medical or diagnostical imaging device, e.g., a microscope, an endoscope, an exoscope, or it can be a device that is configured to exchange information with an imaging device. If the device exchanges information with an image device, the device can be a network device and in particular be in communication with a plurality of imaging devices. It can be a separate device.

Operational data for an imaging device, such as a microscope, may comprise information describing how, when, and under what conditions the device is used and/or data that is generated of the imaging device during operation, such as image data. Operational data may comprise in particular configuration data like magnification, illumination settings, exposure times, and/or filter selections. Operational data may also comprise system status details, such as temperature, humidity, error logs, and/or calibration records. Usage logs document user interactions, session timelines, and/or software modifications may also be comprised by operational data. Additionally, network logs can track data transfers and connectivity status. Furthermore, performance metrics monitor scanning rates, diagnostic checks, and processing times for images. Additionally or alternatively, operational data may comprise image information, experimental information, image-based information, and/or workflow information.

Instruction data can be any data that is generated based on operational data. Therefore, operational data may be processed, in particular summarized and/or extended by an artificial intelligence system such as a large language model (LLM). Instruction data can be human-readable data. This can in particular be obtained by using an LLM to transform operational data to human-readable information. Instruction data may comprise operational data. Additionally instruction data may comprise user information.

Post-operational data can be data that describes the operation of the imaging device. Post-operational data can in particular be human-readable information, such as text. Additionally or alternatively, post-operational data may comprise machine-readable information, such as control and/or configuration information for the imaging device and/or for another imaging device.

By analyzing operational data to derive instruction data and - based on the instruction data - instruct an artificial intelligence network to process the operational data further, post-operational data can be derived that can either describe the post-operational data more specifically and/or in a specific context (e.g. with respect to a pre-defined illness), or machine data can be derived to operate further the device and or other devices, e.g. imaging devices.

An embodiment of the first aspect is related to a device for generating post-operational data of an imaging device,
*configured to:*
- *obtain an output template information;*
*wherein the post-operational data set is based on the obtained output template information.*

Output template information may refer to a structured representation of post-operational data within a standardized format and/or a standardized information content, ensuring consistent organization and facilitating seamless further processing of the post-operational data by humans and/or machines. By defining fields, sections, and data types, an output template may clarify a meaning of each piece of information. This may simplify subsequent processing or integration into larger systems. For example, JSON, XML, and/or YAML templates may be used to structure machine-readable data and in particular incorporate metadata, usage logs, environmental parameters, and/or relevant operational data details in discrete, easily accessible blocks. Because these blocks are labeled, software applications can parse them for subsequent tasks such as analytics, reporting, or troubleshooting. Additionally or alternatively, human operators may benefit from easily navigable outputs, so they can locate significant insights quickly. HTML, docx, MD, and/or structured text may be used to structure human readable post-operational data.

Output template information may act as a blueprint that not only ensures consistency across multiple outputs but also fosters transparency and efficiency in data handling, improving overall system reliability. Moreover, well-structured templates ensure minimal ambiguity, preventing misinterpretation of critical data fields. Output template information may be based on one or more pre-defined templates. Output template information may be provided by a human user. Additionally or alternatively, output template information may be selected by an artificial intelligence.

In particular for control purposes, post-operational data can also be provided via a pre-defined, in particular standardized, communication protocol, and/or a bus system, e.g. a CAN bus system. In this way the device or other devices may be effectively configured and/or controlled for further operations.

Output template information may not only be provided for the artificial intelligence network that directly produces the post-operational data. Output template information may be provided for any artificial intelligence model involved in generating the post-operational data.

An embodiment of the first aspect is related to a device for generating post-operational data of an imaging device,
*wherein the operational data comprises one or more of the following types:*
- *image data ;*
- *imaging device parameter data ;*
- *imaging device environmental data* ;
- *image-processing data ;*
- *operational information of one or more earlier operations of the imaging device and*/*or of another imaging device;*
- *workflow information of one or more earlier operations of the imaging device and*/*or of another imaging device.*

Operational data for imaging devices such as microscopes or endoscopes may comprise information that captures how, when, and under what conditions the device is used. Operational data of an imaging device can be Image data. Image data can in particular be image data of a sample observed with the imaging device. Additionally or alternatively, operational data may comprise Imaging device parameter data. Hence, operational data may comprise any information that describes the imaging device during imaging a sample, such as aperture of a microscope, light intensity and spectral distribution with which the sample is observed. Additionally or alternatively, operational data may comprise information like magnification, viewing angle, illumination settings, exposure times, frame rates, and/or any filters applied. Operational data may also comprise information related to environmental conditions (e.g., temperature, humidity), device health indicators (e.g., error logs, maintenance schedules), and usage logs (e.g., timestamps of procedures, who operated the device, software command histories). Additionally or alternatively, operational data may comprise performance metrics, such as scan rates, throughput, and/or processing times. Operational data may comprise all information present or generated during routine or specialized use of an imaging device.

Image processing data may comprise information related to an image, such as one or more parameters for filters, thresholding, segmentation, and morphological operations, brightness, contrast, and/or color mapping settings. Image-processing data may also comprise alignment and/or registration details for combining images. Image processing data may also relate to the content of the image, such as quantity and quality-related information on cells and/or identified tissue structures.

Workflow information may relate to how images are captured, stored, reviewed, and/or reported. This may comprise user tasks, standard protocols, data handling (storage and transfer), interpretation, and/or reporting steps.

An embodiment of the first aspect is related to a device for generating post-operational data of an imaging device,
*configured to:*
- *obtain a user information ; and*
*wherein the instruction data and*/*or the post-operational data set is further based on the user information.*

User information may be used in order to derive instruction data. Additionally or alternatively, user information may be used after a first instruction data set has been computed. The user data may be used to add additional information to the instruction data set. Additionally or alternatively, user information may be used to amend/improve the first instruction data set or further instruction data sets. The user data may be obtained by a large language model as a function of the device.

Additionally or alternatively, user data may be provided after the instruction data has been processed. For example, user data may be provided in order to amend/improve a first set of post-operational data. This data can in particular provided, by an LLM, to a user such that it can be understood by a human. The human user provides information also to the LLM. Based on the user information, the first set of post-operational data is then further curated before it is outputted to a user and/or to a machine.

Integrating user-provided data from an LLM into an AI system according to the first aspect of this disclosure, may involve structured processes. A pre-defined data format may be used as input structures such that the LLM provides structures outputs (JSON, XLM, etc.) with required fields. Also without a structured input format the LLM may provide a structured output, in particular if this is requested in the user prompt. The LLM's outputs may be routed to various modules and/or microservices that handle specialized tasks like search, analytics, or recommendations and/or that process context-specific. This will be explained in more detail later with respect to context-specific processing.

If certain user inputs or model outputs appear uncertain or unclear to the LLM, the device may trigger a validation process that may comprise a verifying information, e.g. one or more questions, to the user. Through careful prompt design, validation layers, and robust orchestration, organizations may transform raw user requests into actionable insights, powering advanced AI solutions that respond accurately, securely, and effectively to evolving needs. Furthermore, structured logging and continuous feedback loops reinforce reliability across deployments.

An embodiment of the first aspect is related to a device for generating post-operational data of an imaging device,
*configured to:*
- *analyze the obtained user information with respect to a pre-defined user information requirement* ;
- *request the user to input further user information , if it was analyzed that a pre-defined information does not fulfill the user information requirement.*

In this way a user information provided to a device according to the first aspect may be validated. In this way a user can be guided to provide sufficient information to an AI-device according to the first aspect of this disclosure.

An LLM can be used to validate and gather user inputs. By pre-defined rules in system or a prior message, the LLM can parse the user's response, check adherence to the pre-defined rules, and request clarifications if data is missing or invalid. This can in particular be provided if a user input is to be provided according to a specific input template type (structured maybe in docx, JSON, YAML, etc.). This iterative approach allows the LLM to collect information piece by piece and improve an information base from which further inquiries and/or data processing is started. Additionally or alternatively, a validation may further combine LLM-driven conversations with back-end validators (which can be a further LLM trained with respect to potential failures and/or missing information, in particular based on one or more input template information) and convey any error or missing-information messages back to the LLM.

An embodiment of the first aspect is related to a device for generating post-operational data of an imaging device,
*configured to one or both of the following:*
- *provide the instruction data to a plurality of artificial intelligence models , wherein each is trained with one type of operational data ; further configured to:*
   - *integrate the output of the artificial intelligence models , in particular by a summary artificial intelligence model, to determine the post-operational data .*

The plurality of context-specific artificial intelligence models (also called "agents" within this disclosure) can comprise one or more LLMs or other machine learning models such as other transformer-based neural networks, or multi-layer perceptrons, recurrent neural networks, convolutional neural networks, autoencoders, and/or generative models. A model type may be based on its function, e.g. an artificial intelligence model for image processing may be based on a convolutional neural network that may be configured in particular as a regressor and/or as a categorizer. Each of the different models may be trained with respect to their function, e.g. the model for image processing may be trained with images and/or image structures.

After the outputs of the different artificial intelligence models has been generated, some or all of the generated information may be integrated by an additional artificial intelligence model. This summary artificial model may be an LLM or at least comprise an LLM. This LLM may obtain further information on how its output, e.g. post-operational data, may be structured. This can be done, as described above, in particular by providing an output template information, e.g. based on one or more JSON, XLM, docx, YAML files, or a pre-defined communication protocol and/or bus system.

An embodiment of the first aspect is related to a device for generating post-operational data of an imaging device,
*configured to:*
- *provide the instruction data to a first artificial intelligence model that identifies one or more context information of the operational data and*/*or of the instruction data;*
- *provide the identified context information to a plurality of second artificial intelligence models , that process the operational data and*/*or the instruction data with respect to one or more of the identified contexts.*

The first artificial intelligence model helps to understand the different contexts that could be analyzed. Therefore, the output information of the first artificial intelligence model may be based on a pre-defined output template. Such a template can comprise structured information, in particular as described earlier, e.g. based on JSON, YAML, XML, and/or docx information. Based on a template information pre-defined contexts may be provided and first the artificial intelligence evaluates which contexts (of the pre-defined contexts) are comprised in the operation data and/or the instruction data.

Based on the different contexts one or more second artificial intelligence models may be activated. The activated second artificial intelligence models may be drawn from plurality of pre-configured second artificial intelligence models. The different second models may be trained with different contexts, e.g. image processing, sample analysis, workflow analysis, imaging device analysis, etc. The activated second artificial intelligence models may be activated based on a user input and/or automatically, in particular based on the identified contexts of the operational data and/or the instruction data. This might comprise information that had been provided by a user before the instruction data was generated.

The identified contexts may be provided to a user and or an LLM for verification. This can be done in particular as described earlier. An artificial intelligence model (e.g. an LLM) may be configured to check the identified contexts for missing information or non-supported contexts. In case it is determined that a certain information is lacking, a user may be involved by an appropriate user output in order to provide more information on the relevant context information.

An embodiment of the first aspect is related to a device for generating post-operational data of an imaging device,
*wherein one or more of the artificial intelligence models is a language model.*

A device according to the first aspect of this disclosure can comprise a plurality of LLMs. LLMs can in particular be used when information is to be obtained by a user and/or when information is to be intended for a user. For example, instruction data may be verified before it is provided to one or more agents. This verification may be governed by an LLM. Additionally or alternatively, one or more of the agents may be an LLM. Such an LLM can then provide information directly readable by a user. Such information can, e.g. be provided as post-operational data and/or as data based on which a user can verify an output of one or more agents before it is provided for determining post-operational data.

An embodiment of the first aspect is related to a device for generating post-operational data of an imaging device,
*wherein the post-operational data set depends on one or more artificial intelligence models configured to be fed with the instruction data, the operational data, or data based on the preceding two data types; and configured to:*
- *provide information from one or more of the artificial intelligence models to a data base , in particular to a system for retrieval augmented generation;*
   *and*/*or*
- *in response to the provision of information, obtain information from the data base for use in one or more of the artificial intelligence models .*

Based on a data base, in particular a retrieval-augmented generation (RAG), an artificial intelligence model's responses may be improved by providing relevant information from an external knowledge source, in particular at inference time. Therefore, the artificial intelligence model sends query information to a data base and receives back information from the data base.

Additionally or alternatively, a query to a data base may be provided by a different artificial intelligence model than to which the response of the data base is send. For example, a query may be sent to a data base from a first artificial intelligence model that has identified one or more contexts of an operational data and/or of an instruction data. Based on the queried context information, the data base will then send additional information to the one or more second artificial intelligence models (agents) that analyze the operational data and/or the instruction data based on one or more of the provided contexts (as described above). **In** case one or more of the second, context-specific artificial intelligence models are not **LLMs,** the information from the data base (which may be human readable information such as scientific papers and/or information gathered from previous experiments) may be adapted by an **LLM** in order to be provided to the respective context-specific second artificial intelligence model.

By dynamically basing model outputs on information of a database, a device according to the first aspect of this disclosure can generate post-operational data that is both contextually relevant and up to date.

An embodiment of the first aspect is related to a device for generating post-operational data of an imaging device,
*wherein the post-operational data depends on a first artificial intelligence model that is configured to a identify one or more context information of the operational data and*/*or the instruction data; and*
*configured to:*
   - *provide information by the first artificial intelligence model to a data base , in particular a RAG system;*
   - *in response to the provision of information, obtain first information from the data base; and*
   - *identify the one or more contexts based on the obtained first information; and*/*or*
*wherein the post-operational data set depends on one or more second artificial intelligence models that provide output information based on one or more identified contexts; and*
*configured to:*
   - *provide information by one or more of the artificial intelligence models to a data base , in particular a RAG system;*
   - *in response to the provision of information, obtain second information from the data base ; and*
   - *provide output information, in particular post-operational data , based on the obtained second information.*

In the first alternative of the embodiment described above, the artificial intelligence model for providing context information is supported by data base information. This can be done in particular during processing time.

Context information may relate to any context an operation and/or operational data of an imaging device may have. A context information may be related to an image context, which is related to all information related to a generated image during an operation of an imaging device (e.g. 3D/2D+T, resolution, number of channels, bit-depth). A context information may be related to an experimental context, which is related to all information related to an experiment and/or an analysis conducted during an operation of an imaging device (e.g. number of channels, markers used for each channel, microscope modality, number of samples, treatment associated to images). A context information may be related to a segmentation context, which is related to all information related to segmentation performed based an image information obtained during an operation of an imaging device and/or other workflow-related information (e.g. information of segmentation algorithms, list of objects and/or measurements computed, history of actions from user in the image analysis software).

In the second alternative of this embodiment, the one or more agents that provide context-specific information are supported by data base information. This can be done in particular also during processing time, i.e. after an agent has received input information and performs output processing.

An embodiment of the first aspect is related to a device for generating post-operational data of an imaging device,
*configured to:*
- *provide the instruction data to a user interface* ;
- *obtain further user information from the user interface;*
- *adapt the instruction data based on the further user information.*

This can be done for the purpose of verification of the instruction data set. Additionally or alternatively, a user can be requested to provide additional information to the instruction data set. In case the instruction data set is not human-readable, an LLM may be involved that transforms the instruction data set to a human readable information before it is presented to gather user feedback information.

A validation and/and or extension of machine-generated information can also be done for the other artificial intelligence models comprised by the device. In case such an artificial intelligence model is not an LLM, the information to be validated/extended may be processed beforehand by an LLM such that it can be interpreted by a user. This LLM may also be used to transform the reflected user information back to the "language" of the artificial intelligence model.

An embodiment of the first aspect is related to a device for generating post-operational data of an imaging device,
*wherein the post-operational data set comprises one or more of:*
- *imaging device parameters;*
- *sample parameters.*

Imaging device parameters may include both metadata (for example, time stamps, camera identifiers, or versioning information) and/or control data (in particular to control an imaging device in realtime) and/or configuration data (such as operational settings and system configurations). These parameters can be incorporated into a text-based overview, such as a human-readable report. Post-operative data can comprise an imaging device's (e.g. a microscope's) parameters (like magnification levels and/or lens configurations), acquisition settings (e.g., exposure time, lighting, or wavelength), and/or analysis parameters (such as image processing algorithms or quantitative measurement thresholds). Storing or presenting these various parameters in a coherent, language-focused document may support more seamless sharing, interpretation, and/or re-use of the imaging results.

An embodiment of the first aspect is related to a device for generating post-operational data of an imaging device,
*configured to:*
- *obtain a further user information* ;
- *adapt the post-operational parameter set based on the further user information .*

Also the post-operational data can be verified and/or extended by user information. This can in particular be implemented in the same way as described above.

An embodiment of the first aspect is related to a device for generating post-operational data of an imaging device,
*configured to:*
- *provide the post-operational data set* as *configuration data and*/*or control data for one or both of:*
   -- *an imaging device , in particular the imaging device;*
   -- *an image processing function;*
*in particular together with operational data .*

Having parameter and/or machine-signal information may enable direct control and/or configuration of an imaging device. This may allow users to fine-tune and/or replicate imaging conditions for consistent and optimized data acquisition. The control and/or configuration information can in particular be provided to the imaging device from which the operational data stems from. Additionally or alternatively, the control and/or configuration information may be provided to one or more imaging devices, which differ from the device from which the operational data stems. Control and/or configuration information can in particular be provided in a real-time control loop in order to control the device(s) effectively. Control and/or configuration data may be according to a pre-defined communication protocol and/or in a pre-defined format, such as a bus system (e.g. CAN bus).

A second aspect of the present disclosure is related to a method for generating post-operational data of an imaging device,
*comprising:*
- *obtaining operational data of an imaging device;*
- *determining instruction data based on:*
   -- *the operational data; and*
- *determine a post-operational data set based on:*
   -- *the instruction data; and*
   -- *the operational data.*

A method according to the second aspect can comprise one or more steps to execute one or more functions of a device according to the first aspect of this disclosure. Steps of a method according to the second aspect of this disclosure can be configured such that thy comprise features of a device according to the first aspect of this disclosure.

A further aspect of the present disclosure is related to a computing device comprising a processor configured to carry out the method according to any one of preceding aspects/embodiments.

A further process of the present disclosure is related to a computer program product comprising instructions which, when the program is executed by a computer system, cause the computer system to carry out the method according to any one of the preceding aspects/embodiments.

A further aspect of the present disclosure is related to a computer-readable medium comprising instructions which, when executed by a computer system, cause the computer system to carry out the method according to any one of the preceding aspects/embodiments.

### Brief description of the figures

Further advantages and features result from the following embodiments, some of which refer to the figures. The figures do not always show the embodiments to scale. The dimensions of the various features may be enlarged or reduced, in particular for clarity of description. For this purpose the figures are at least partially schematized.
Fig. 1 illustrates a block diagram of a device according to an embodiment of this disclosure.
Fig. 2 illustrates a block diagram of a device according to an embodiment of this disclosure.
Fig. 3 illustrates a block diagram of a device according to an embodiment of this disclosure.
Fig. 4 illustrates a block diagram of a device according to an embodiment of this disclosure.
Fig. 5 illustrates a microscope system according to or for embodiments of this disclosure.

Although some aspects have been described in the context of an apparatus (or a system) in the present disclosure, the description of these aspects also represents a description of the corresponding method, where a block or device corresponds to a method step or a feature of a method step.

Analogously, aspects described in the context of a method step also represent a description of a corresponding block, item, or feature of a corresponding apparatus or of a system that may in particular be distributed over different locations and is configured to exchange information between the different locations with respective communication means.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

As used herein the term "and/or" includes any and all combinations of one or more of the associated listed items and may be abbreviated as "/". Expressions as, "for example", "e.g.", or "in particular" denote facultative or optional features that can be combined with all other (mandatory, facultative, or optional) features of the aspects or embodiments of this disclosure, until explicitly stated otherwise.

In the following description reference is made to the accompanying figures which form part of the disclosure, and which illustrate specific aspects in which the present disclosure can be understood. Identical reference signs refer to identical or at least functionally or structurally similar features.

### Detailed description

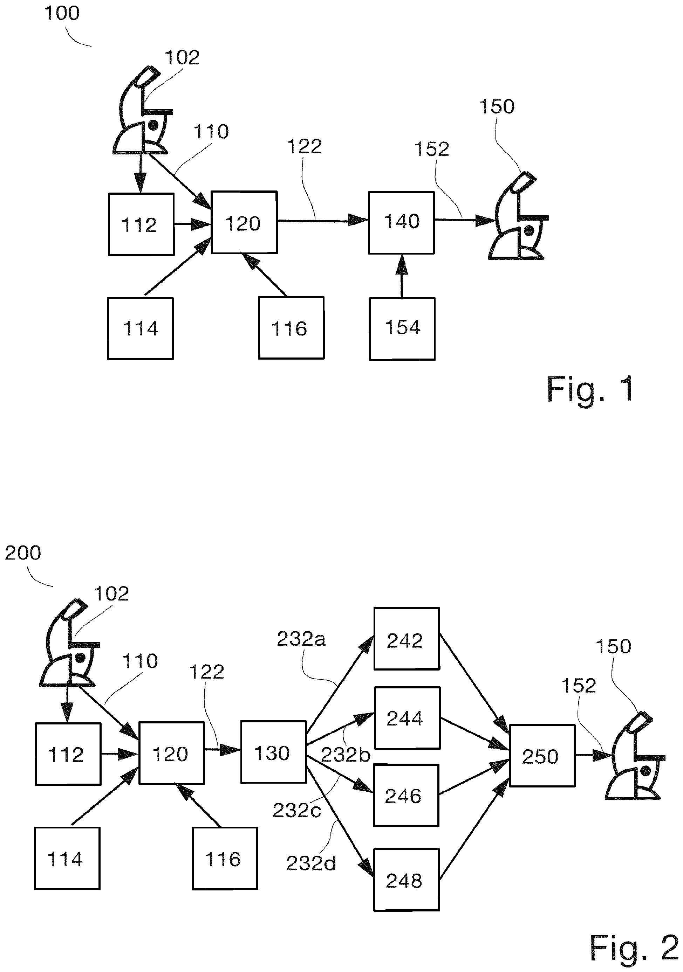

Fig. 1 illustrates a block diagram of a device 100 according to an embodiment of this disclosure. The device obtains operational data 110 from an operational microscope 102. Operational data can comprise any data of the microscope obtained and/or generated during an operation, such as an analysis of a sample. Based on the image data of the device, image processed data 112 is generated. This can be done by the device itself or a third party device or function. Additionally, workflow information 114 is provided, e.g. from an external data base, wherein the workflow information comprises information on how the microscope 102 has been used to achieve the image information 110 of the sample.

The operational information 110, the image-processing information 112, and the workflow information 114 are integrated by the integration function 120, e.g. by structuring them according to a pre-defined information template, such as a JSON file, or by incorporating all information into a single text file. Additionally to these three data types, user information 116 may also be provided over a graphical user interface, wherein the user information may comprise information on an objective of an analysis of the sample. User information may be optional.

Based on the information 110, 112, 114, and 114, an instruction data set 122 generated by the integration function 120. Based on the instruction data 122, which can also be regarded as an extended user prompt, a subsequent artificial intelligence model, in particular a large language model (LLM), is fed. The instruction data 122 is provided to a large LLM 140. The LLM 140 is trained with user cases. These can be real user cases or simulated user cases. The user cases can be provided as reports about the experiment, for example. Furthermore, the LLM 140 is provided with a pre-defined template information 154, which comprises information on how to structure the output information. Based on this information, the LLM140 generates a post-operational data set 152 as output information. The LLM 140 can additionally be provided with requirement information 154 for output information, such that the post-operational data comprises a pre-defined content and/or is structured in a pre-defined way.

The post-operational data set 152 is then communicated to a target microscope 150. The target microscope 150 may be the operational microscope 102 from which the operational data 110 for the actual analysis stems. In this way, the device 100 may analyze operational information 110 in order to adapt an ongoing operation of the microscope 102, 150. Alternatively, the post-operational data set 152 may be used to configure and/or control the microscope 102, 150 for another experiment. Additionally or alternatively, the post-operational data set may be sent to one or more other microscopes for configuration and/or control purposes. Additionally or alternatively, the post-operational data may be configured as a report intended to be read by a human user.

Fig. 2 illustrates a block diagram of a device 200 according to an embodiment of this disclosure. The device 200 comprises functions similar to the device 100 described in relation to the previous figure. In difference to the device 100, the device 200 comprises an context-specific information processing in order to determine post-operational data 152. Therefore, based on the instruction data 122, multiple types of context-specific instruction data is generated, e.g. by artificial intelligence model 130.

For example, image-specific instruction data 232a, microscope-specific instruction data 232b, workflow-specific instruction data 232c, and experiment-specific instruction data 232d are generated by the LLM 130. The context-specific instruction-data sets 232a - 232d are then send to a specifically trained artificial intelligence model 242 - 248 each. The instruction data set 232a is provided to artificial intelligence model 242, which is a convolutional neural network trained to identify specific structures in microscope images. Additionally, the instruction data set 232b is provided to artificial intelligence model 244, which trained to process microscope-specific information. The instruction data set 232c is provided to artificial intelligence model 246, which trained to process workflow-specific information. And the instruction data set 232d is provided to artificial intelligence model 248, which trained to process experiment-specific information. The different artificial intelligence models 242 - 248 can also be called agents, as each model is configured to perform a (context-)specific task. Alternatively, the different agents may also be fed with the full instruction data 122. In this case the context-specific categorization of the instruction data does not take place.

The information generated by the agents 242 - 248 is then provided to a further artificial intelligence model 250. This can be an LLM configured to summarize different context-specific information. The AI-model 250 then generates the post-operational data 152, which is provided to another machine and/or to a human user (e.g. as a readable report).

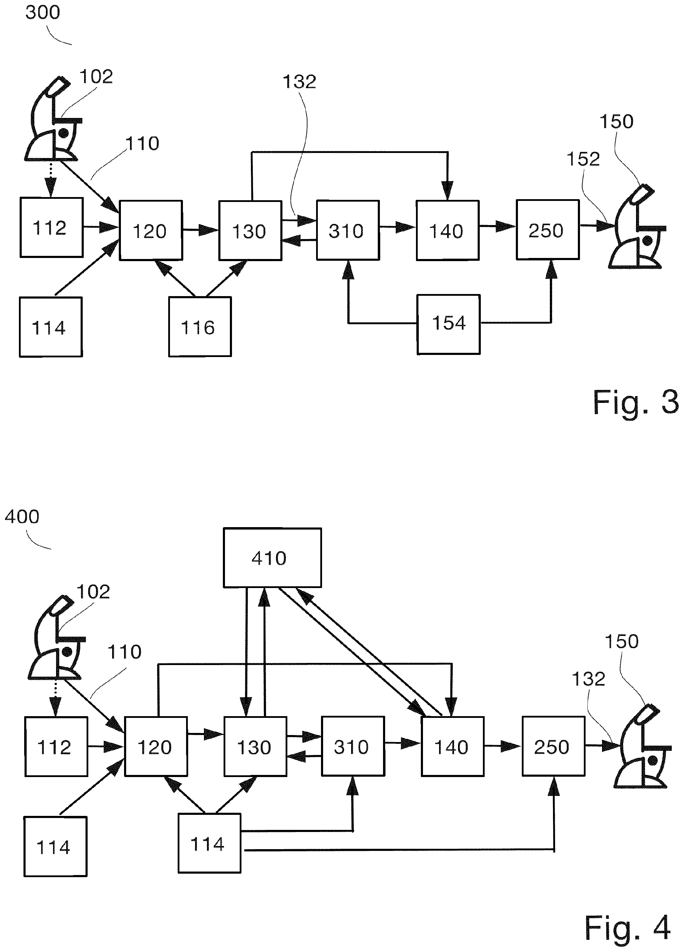

Fig. 3 illustrates a block diagram of a device 300 according to an embodiment of this disclosure. The device 300 comprises functions similar to the devices 100 and/or 200 described in relation to the previous figures with which the device 300 may be fully compatible with. In difference to the devices 100, 200, the device 300 comprises an artificial intelligence model 310, which is configured to validate a plurality of context information provided by the artificial intelligence model 130. The function 310 can also be an integral part of the context categorization 130. The AI-model 310 can be e.g. an LLM, but also other categorizing AI-models are possible, such as a convolutional neural network. The function 310 receives instruction information 132. The validation function 310 may work based on template information 154, wherein the template information may pre-define which contexts the categorizer should identify in the instruction data and/or the operational data. The validation function 310 may also be configured to validate the identified context information and/or suggests further contexts, in particular context information that may not be provided in the context template 154. The suggested one or more further contexts may in particular be provided via a graphical user interface to a user in order to receive additional user information for the context categorization.

The identified contexts are provided to a single multi-context AI-model 140. Alternatively, the AI-system 140 can also comprise a plurality of context-specific AI-models (similar to the models 242 - 248 of the previous figure). Furthermore, the LLM 140 may also receive the whole or a part of the operational information 110, 112, 114, 116, and/or of the instruction data 122. In case of the latter, an optional artificial intelligence model 250 may be used to summarize the different results into a set of post-operational data, in particular based on a pre-defined post-operational data structure 154, which may be provided as JSON, YAML, XML, and/or docx information.

Fig. 4 illustrates a block diagram of a device 400 according to an embodiment of this disclosure. The device 400 comprises functions similar to the devices 100, 200, and/or 300 described in relation to the previous figures with which the device 400 may be fully compatible with. In difference to the devices 100, 200, 300 the device 400 is configured to interface with an external retrieval augmented generation (RAG) system.

The RAG system may be consulted by the LLM 130, for generating the different contexts that are used for the subsequent LLM 140 in order to analyze the instruction data and/or the operational data according to different contexts. In order to identify these contexts from the instruction data 122 and/or from the operational data 110, 112, 114, 116, the LLM 130 may query the RAG-system 410. Based on the query, the RAG system will respond with information that helps the LLM 130 to identify the different contexts which are later used by the LLM 140 to generate the context-specific output data. Alternatively, the identified contexts may be provided to the context-specific agents 242 - 248.

Additionally or alternatively, the RAG system 510 may be consulted for generating context-specific output data and/or post-operational data. The RAG system 510 may also be a different RAG system than the one used for supporting the context generation by the LLM 130. The LLM 140 may query the RAG system with information regarding a specific context, e.g. an image-processing context, and receives back a response comprising additional information for generating an output information.

User information may be provided in order to amend the information provided to the AI-models 130, 310, and/or 250.

Example for output template:

```
 {
 ""title"": ʺʺTITLE_PLACEHOLDERʺʺ,
 ʺʺsummaryʺʺ: ""SHORT_SUMMARY_OF_CONTENT"",
 ""details"": [
  {
    ""heading"": ʺʺSECTION_HEADINGʺʺ,
    ʺʺcontentʺʺ: ʺʺDETAILED_TEXT_UNDER_THIS_SECTIONʺʺ
  },
  {
    ""heading"": ʺʺSECTION_HEADINGʺʺ,
    ʺʺcontentʺʺ: ʺʺDETAILED_TEXT_UNDER_THIS_SECTIONʺʺ
  }
 ],
 ʺʺconclusionʺʺ: ʺʺFINAL_THOUGHTS_OR_SUMMARYʺʺ,
 ""references"": [
  ʺʺREFERENCE_OR_SOURCE_1ʺʺ,
  ʺʺREFERENCE_OR_SOURCE_2ʺʺ
 ]
 }.
 
```

Example for output template:

```
{
 ""microscope_configuration"": {
  ʺʺhardware_infoʺʺ: {
    ""manufacturer"": ""ACME Microscopesʺʺ,
    ""model"": ""X100"",
    ""serial_number"": ""SN123456"",
    ʺʺfirmware_versionʺʺ: ʺʺ1.2.3ʺʺ
  },
  ʺʺimaging_settingsʺʺ: {
    ""magnification"": ʺʺ40xʺʺ,
    ""illumination_intensity"": ʺʺ75%ʺʺ,
    ʺʺexposure_time_msʺʺ: 200,
    ʺʺfilter_selectionʺʺ: ""Green Fluorescent Protein (GFP)ʺʺ,
    ʺʺcamera_gainʺʺ: 1.0
  },
  ʺʺenvironmental_parametersʺʺ: {
    ʺʺtemperature_celsiusʺʺ: 25.0,
    ʺʺhumidity_percentʺʺ: 45.0
  },
  ʺʺsession_infoʺʺ: {
    ʺʺoperator _nameʺʺ: ""Jane Doeʺʺ,
    ʺʺtimestamp_startʺʺ: ʺʺ2025-03-07T10:00:00Zʺʺ,
    ""session_id"": ""abcdef-12345"",
    ""notes"": ""Focusing on sample #42ʺʺ
  },
  ""calibration"": {
    ʺʺlast_calibration_dateʺʺ: ʺʺ2025-02-15T09:30:00Zʺʺ,
    ʺʺcalibration_logsʺʺ: [
     ""Calibrated at 10x magnification"",
     ʺʺChecked field uniformityʺʺ
    ]
  }
 }
 }.
```

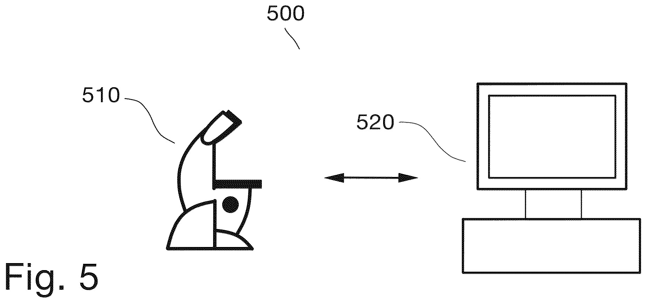

Some embodiments relate to a microscope comprising a system as described in connection with one or more of the Figs. 1 to 4. Alternatively, a microscope may be part of or connected to a system as described in connection with one or more of the Figs. 1 to 4. Fig. 5 shows a schematic illustration of a system 500 configured to perform a method described herein. The system 500 comprises a microscope 510 and a computer system 520. The microscope 510 is configured to take images and is connected to the computer system 520. The computer system 520 is configured to execute at least a part of a method described herein. The computer system 520 may be configured to execute a machine learning algorithm. The computer system 520 and microscope 510 may be separate entities but can also be integrated together in one common housing. The computer system 520 may be part of a central processing system of the microscope 510 and/or the computer system 520 may be part of a subcomponent of the microscope 510, such as a sensor, an actor, a camera or an illumination unit, etc. of the microscope 510.

The computer system 520 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 520 may comprise any circuit or combination of circuits. In one embodiment, the computer system 520 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system 520 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 520 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 520 may also include a display device, one or more speakers, and a keyboard and/or controller, which can include a mouse, trackball, touch screen, voice-recognition device, or any other device that permits a system user to input information into and receive information from the computer system 520.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of reference signs

- 100: device
- 102: operational microscope
- 110: operational data
- 112: image-processed data
- 114: workflow information
- 116: user information
- 120: integration function
- 122: instruction data
- 130: context generator
- 140: LLM
- 150: target microscope
- 152: post operational data set
- 154: output requirements
- 200: device
- 232a: image specific instruction data
- 232b: microscope specific instruction data
- 232c: workflow specific instruction data
- 232d: experiment specific instruction data
- 242: image specific AI model
- 244: microscope specific AI model
- 246: workflow specific AI model
- 248: experiment specific AI model
- 250: summarizing LLM
- 300: device
- 310: context validation
- 400: device
- 410: RAG system
- 500: system
- 510: microscope
- 520: computer system

## Claims

1. A device for generating post-operational data of an imaging device, configured to:
- obtain operational data (110, 112, 114) of an imaging device;
- determine instruction data (120) based on:
- - the operational data; and
- determine a post-operational data set (152) based on:
-- the instruction data; and
-- the operational data.

2. The device according to the preceding claim,
configured to:
- obtain an output template information (154);
wherein the post-operational data set (152) is based on the obtained output template information.

3. The device according to the preceding claim,
wherein the operational data comprises one or more of the following types:
- image data (110);
- imaging device parameter data (110);
- imaging device environmental data (110);
- image-processing data (112);
- operational information of one or more earlier operations of the imaging device and/or of another imaging device;
- workflow information (114) of one or more earlier operations of the imaging device and/or of another imaging device.

4. The device according to one of the preceding claims,
configured to:
- obtain a user information (116); and
wherein the instruction data (122) and/or the post-operational data set (152) is further based on the user information.

5. The device according to the preceding claims,
configured to:
- analyze the obtained user information (116) with respect to a pre-defined user information requirement (154);
- request the user to input further user information (114), if it was analyzed that the pre-defined information does not fulfill the user information requirement.

6. The device according to one of the preceding claims,
configured to one or both of the following:
- provide the instruction data (122) to a plurality of artificial intelligence models (242, 244, 246, 248), wherein each is trained with one type of operational data (110);
further configured to:
- integrate the output of the artificial intelligence models (242, 244, 246, 248), in particular by a summary artificial intelligence model (250), to determine the post-operational data (152).

7. The device according to one of the preceding 2 - 5 claims,
configured to:
- provide the instruction data (122) to a first artificial intelligence model (130) that identifies one or more context information of the operational data and/or of the instruction data;
- provide the identified context information to a plurality of second artificial intelligence models (140, 242, 244, 246, 248), that process the operational data and/or the instruction data with respect to one or more of the identified contexts.

8. The device according to the preceding claim,
wherein one or more of the artificial intelligence models (130, 140, 242, 244, 246, 248, 250) is a language model.

9. The device according to one of the preceding claims,
wherein the post-operational data set (152) depends on one or more artificial intelligence models (130, 140, 242, 244, 246, 248, 250) configured to be fed with the instruction data, the operational data, or data based on the preceding two data types; and
configured to:
- provide information from one or more of the artificial intelligence models to a data base (410), in particular to a system for retrieval augmented generation;
and/or
- in response to the provision of information, obtain information from the data base (410) for use in one or more of the artificial intelligence models (130, 140, 242, 244, 246, 248, 250).

10. The device according to one of the preceding claims,
wherein the post-operational data (152) depends on a first artificial intelligence model (130) that is configured to a identify one or more context information of the operational data and/or the instruction data; and
configured to:
- provide information by the first artificial intelligence model to a data base (410), in particular a RAG system;
- in response to the provision of information, obtain first information from the data base; and
- identify the one or more contexts based on the obtained first information;
and/or
wherein the post-operational data set (152) depends on one or more second artificial intelligence models (140, 242, 244, 246, 248, 250) that provide output information based on one or more identified contexts; and
configured to:
- provide information by one or more of the artificial intelligence models (140, 242, 244, 246, 248, 250) to a data base (410), in particular a RAG system;
- in response to the provision of information, obtain second information from the data base (410); and
- provide output information, in particular post-operational data (152), based on the obtained second information.

11. **The** device according to one of the two preceding claims,
configured to:
- provide the instruction data (122) to a user interface (116);
- obtain further user information from the user interface;
- adapt the instruction data based on the further user information.

12. **The** device according to one of the preceding claims,
wherein the post-operational data set (152) comprises one or more of:
- imaging device parameters;
- sample parameters.

13. **The** device according to one of the preceding claims,
configured to:
- obtain a further user information (116);
- adapt the post-operational parameter set (152) based on the further user information (116).

14. **The** device according to one of the preceding claims,
configured to:
- provide the post-operational data set (152) as configuration data and/or control data for one or both of:
- - an imaging device (150), in particular the imaging device;
- - an image processing function;
in particular together with operational data (110).

15. A computer-implemented method for generating post-operational data from an imaging device,
comprising
- obtaining operational data (110, 112, 114) of an imaging device;
- determining instruction data (122) based on:
- - the operational data; and
- determine a post-operational data set (152) based on:
-- the instruction data; and
-- the operational data.
